(19) Europäisches Patentamt
European Patent Office
Office européen des brevets

(11) EP 4 446 292 A1

(12) EUROPEAN PATENT APPLICATION

(43) Date of publication:
**16.10.2024 Bulletin 2024/42**

(21) Application number: **24150092.5**

(22) Date of filing: **02.01.2024**

(51) International Patent Classification (IPC):
***C03C 17/32*** (2006.01) ***B32B 17/10*** (2006.01)
***C03C 17/34*** (2006.01) ***C09D 133/14*** (2006.01)
***G01N 33/00*** (2006.01)

(52) Cooperative Patent Classification (CPC):
**C03C 17/324; B32B 17/10; C03C 17/3405; C09D 133/14; G01N 33/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.04.2023 JP 2023066206**

(71) Applicant: **Sumitomo Rubber Industries, Ltd.**
**Kobe-shi, Hyogo 651-0072 (JP)**

(72) Inventors:
- **MINAGAWA, Yasuhisa**
**Kobe-shi, Hyogo, 651-0072 (JP)**
- **DOKAI, Chiaki**
**Kobe-shi, Hyogo, 651-0072 (JP)**

(74) Representative: **Manitz Finsterwald**
**Patent- und Rechtsanwaltspartnerschaft mbB**
**Martin-Greif-Strasse 1**
**80336 München (DE)**

# (54) POLYMER-COATED GLASS SUBSTRATE

(57) Provided is a polymer-coated glass substrate having a surface with controlled irregularity and a low elastic modulus. The polymer-coated glass substrate includes a glass substrate and two or more hydrophilic polymer layers on a surface of the glass substrate.

EP 4 446 292 A1

**Description**

TECHNICAL FIELD

The present invention relates to a polymer-coated glass substrate.

BACKGROUND ART

A technique of coating the surface of a glass substrate with a special polymer has been proposed to prepare a device for capturing specific cells (e.g., blood cells, cancer cells present in blood or biological fluid) from blood or biological fluid.

However, coating with some special polymers is difficult to form a flat smooth surface. Since irregularity of the surface affects the capture of specific cells, a substrate which has a surface with controlled irregularity to be excellent in, for example, capturing specific cells such as cancer cells is desired (see, Patent Literature 1). Moreover, a large surface irregularity is likely to cause white turbidity of the coating surface. Therefore, the irregularity of the surface is desirably controlled to suppress white turbidity of the coating surface. Suppression of white turbidity of the coating surface is expected to improve capture, etc. of specific cells such as cancer cells.

CITATION LIST

PATENT LITERATURE

Patent Literature 1: JP 2005-523981 T

SUMMARY OF INVENTION

TECHNICAL PROBLEM

The present invention aims to solve the above problem and provide a polymer-coated glass substrate having a surface with controlled irregularity and a low elastic modulus.

SOLUTION TO PROBLEM

The present invention relates to a polymer-coated glass substrate including a glass substrate and two or more hydrophilic polymer layers on a surface of the glass substrate.

ADVANTAGEOUS EFFECTS OF INVENTION

The polymer-coated glass substrate according to the present invention includes a glass substrate and two or more hydrophilic polymer layers on a surface of the glass substrate. Thus, the present invention can provide a polymer-coated glass substrate having a surface with controlled irregularity and a low elastic modulus. The polymer-coated glass substrate exhibits an effect of improving capture of specific cells such as cancer cells.

BRIEF DESCRIPTION OF DRAWINGS

FIG. 1 shows exemplary pictures of glass substrates produced in Example 4 and Comparative Example 1.

DESCRIPTION OF EMBODIMENTS

The present invention relates to a polymer-coated glass substrate including a glass substrate and two or more hydrophilic polymer layers on a surface of the glass substrate. With the two or more hydrophilic polymer layers on the surface of the glass substrate, the irregularity of the substrate surface can be controlled, whereby it is possible to provide a polymer-coated glass substrate coated with a hydrophilic polymer layer having a low surface roughness, a high flat smoothness, and a low surface elastic modulus.

The number of tumor cells (cancer cells, etc.) appearing in biological fluid, such as circulating tumor cells (several to hundreds of cells/1 ml of blood), is very small, and it is considered important to capture tumor cells present in the sampled biological fluid as many as possible to analyze them. The polymer-coated glass substrate of the present invention includes two or more hydrophilic polymer layers on a glass substrate, thereby forming a hydrophilic polymer

layer having a surface with controlled irregularity, a low roughness, and a high flat smoothness. The surface of the formed hydrophilic polymer layer has a low elastic modulus. The irregularity of the hydrophilic polymer layer affects the capture of specific cells such as cancer cells. The surface with controlled irregularity and a high flat smoothness is excellent in capturing specific cells. The elastic modulus of the surface affects the capture of specific cells. The lower the elastic modulus is, the more the capture is improved. Therefore, for example, the polymer-coated glass substrate is usable to determine the number of tumor cells in biological fluid to evaluate the cancer-treating effect by capturing tumor cells with the two or more hydrophilic polymer layers on the surface of the glass substrate and counting the number of captured tumor cells. Moreover, the captured tumor cells may be cultured and then used to determine the effects of drugs such as anticancer drugs. Thus, the effects of drugs such as anticancer drugs ex vivo can be confirmed before administration. This technique is also usable to screen drugs such as anticancer drugs.

The type of glass in the polymer-coated glass substrate is not limited. Examples include soda lime glass, alkali-free glass, borosilicate glass ($SiO_2$-$B_2O_3$-ZnO glass, $S_1O_2$-$B_2O_3$-$B_{12}O_3$ glass, etc.), potash glass, crystal glass (glass containing PbO, such as $SiO_2$-PbO glass, $SiO_2$-PbO-$B_2O_3$ glass, $SiO_2$-$B_2O_3$-PbO glass, etc.), titanium crystal glass, barium glass, boron glass ($B_2O_3$-ZnO-PbO glass, $B_2O_3$-ZnO-$Bi_2O_3$ glass, $B_2O_3$-$Bi_2O_3$ glass, $B_2O_3$-ZnO glass, etc.), strontium glass, alumina silicate glass, soda-zinc glass, and soda-barium glass (BaO-$SiO_2$ glass, etc.). These types of glass may be used alone or in admixtures of two or more.

Although the thickness of the glass substrate is not limited, the average thickness is preferably not less than 100 um and not more than 3000 um, more preferably not less than 100 um and not more than 2000 um. Here, the term "average thickness" refers to the average of the thicknesses measured at ten arbitrary points using a micrometer.

In the polymer-coated glass substrate, two or more hydrophilic polymer layers are formed on a surface of the glass substrate. The two or more hydrophilic polymer layers may include the same hydrophilic polymer or different hydrophilic polymers. In order to better achieve the advantageous effect, the two or more hydrophilic polymer layers desirably include the same hydrophilic polymer. The hydrophilic polymer in the hydrophilic polymer layers may be an appropriate known hydrophilic polymer.

The hydrophilic polymer in each of the two or more hydrophilic polymer layers is not limited and may be an appropriate hydrophilic polymer, for example. The hydrophilic polymer may be used alone or in combinations of two or more.

The hydrophilic polymer can be produced by known methods. For example, it may be synthesized by polymerizing a hydrophilic monomer of the target hydrophilic polymer by a known method using a solution of the hydrophilic monomer. Non-limiting examples of the solvent of the hydrophilic monomer solution include those described later. Toluene or methanol is preferred among these.

Examples of the hydrophilic polymer include homopolymers or copolymers of one or two or more hydrophilic monomers, and copolymers of one or two or more hydrophilic monomers and one or two or more different monomers. These may be used alone or in combinations of two or more.

Non-limiting examples of the hydrophilic monomer include various monomers containing hydrophilic groups. Examples of the hydrophilic groups include known hydrophilic groups such as an amide group, a sulfuric acid group, a sulfonic acid group, a carboxylic acid group, a hydroxy group, an amino group, and an oxyethylene group.

Specific examples of the hydrophilic monomer include (meth)acrylic acids; (meth)acrylic acid esters; alkoxyalkyl (meth)acrylates such as methoxyethyl (meth)acrylates; hydroxyalkyl (meth)acrylates such as hydroxyethyl (meth)acrylates; (meth)acrylamides; and (meth)acrylamide derivatives containing cyclic groups such as (meth)acryloylmorpholines. Of these, (meth)acrylic acids, (meth)acrylic acid esters, alkoxyalkyl (meth)acrylates, and (meth)acrylamide derivatives containing cyclic groups are preferred; alkoxyalkyl (meth)acrylates and (meth)acryloylmorpholines are more preferred; alkoxyalkyl (meth)acrylates are still more preferred; and 2-methoxyethylacrylate is particularly preferred. These may be used alone or in combinations of two or more.

The different monomer may be appropriately selected as long as it does not inhibit the effect of the hydrophilic polymer. Specific examples of the different monomer include aromatic monomers such as styrene, vinyl acetate, and N-isopropylacrylamide which can impart temperature responsiveness. These may be used alone or in combinations of two or more.

Specific examples of the homopolymers and copolymers include: homopolymers formed from a single hydrophilic monomer, such as polyacrylic acids, polyacrylic acid esters, polymethacrylic acids, polymethacrylic acid esters, polyacryloylmorpholines, polymethacryloylmorpholines, polyacrylamides, polymethacrylamides, polyalkoxyalkyl acrylates, and polyalkoxyalkyl methacrylates; copolymers formed from two or more hydrophilic monomers listed above; and copolymers formed from one or more hydrophilic monomers listed above and one or more different monomers listed above. These hydrophilic polymers may be used alone or in combinations of two or more.

The hydrophilic polymer is preferably at least one selected from the group consisting of hydrophilic polymers represented by the following formula (I):

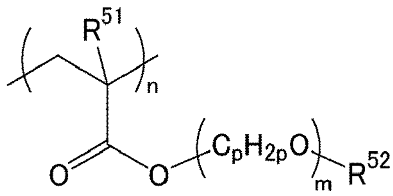

(I)

wherein $R^{51}$ represents a hydrogen atom or a methyl group; $R^{52}$ represents an alkyl group; p represents 1 to 8; m represents 1 to 5; and n represents the number of repetitions. These may be used alone or in combinations of two or more.

Suitable examples of the hydrophilic polymers represented by the formula (I) include hydrophilic polymers represented by the following formula (I-1):

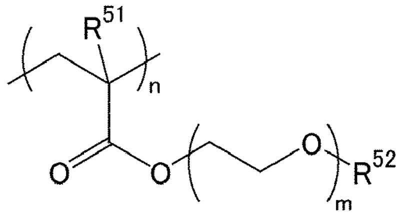

(I-1)

wherein $R^{51}$ represents a hydrogen atom or a methyl group; $R^{52}$ represents an alkyl group; m represents 1 to 5; and n represents the number of repetitions. These may be used alone or in combinations of two or more.

The number of carbon atoms of the alkyl group for $R^{52}$ is preferably 1 to 10, more preferably 1 to 5. $R^{52}$ is particularly preferably a methyl group or an ethyl group. The symbol p is preferably 1 to 5, more preferably 1 to 3. The symbol m is preferably 1 to 3. The symbol n (the number of repeating units) is preferably 15 to 1500, more preferably 40 to 1200.

Suitable examples of the hydrophilic polymer also include copolymers of at least one hydrophilic monomer selected from the group consisting of compounds represented by the following formula (II) with a different monomer. These may be used alone or in combinations of two or more.

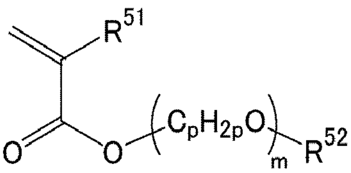

(II)

In the formula, $R^{51}$ represents a hydrogen atom or a methyl group; $R^{52}$ represents an alkyl group; p represents 1 to 8; and m represents 1 to 5.

The compounds represented by the formula (II) are suitably at least one selected from the group consisting of compounds represented by the following formula (II-1). These may be used alone or in combinations of two or more.

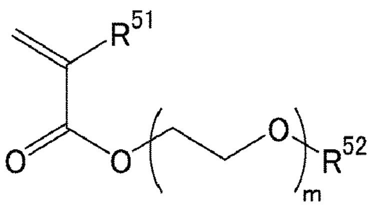

(II-1)

In the formula, $R^{51}$ represents a hydrogen atom or a methyl group; $R^{52}$ represents an alkyl group; and m represents 1 to 5.

In order to form a hydrophilic polymer layer having a low surface roughness and a high flat smoothness by controlling the irregularity of the substrate surface, the hydrophilic polymers represented by the formula (I) are preferred, and the hydrophilic polymers represented by the formula (I-1) are particularly preferred among the above-described hydrophilic polymers.

In order to better achieve the advantageous effect, the number average molecular weight (Mn) of the hydrophilic

polymer in each of the two or more hydrophilic polymer layers is preferably 5000 or more, more preferably 10000 or more, still more preferably 15000 or more, while it is preferably 200000 or less, more preferably 150000 or less, still more preferably 120000 or less.

In order to better achieve the advantageous effect, among the two or more hydrophilic polymer layers, preferably, the hydrophilic polymer in the hydrophilic polymer layer on the surface of the glass substrate (the hydrophilic polymer layer formed to contact the glass substrate (hereinafter, also referred to as innermost layer)) has a number average molecular weight equal to or higher than that of the hydrophilic polymer in other hydrophilic polymer layers (one or more hydrophilic polymer layers among the two or more hydrophilic polymer layers excluding the innermost layer). More preferably, the hydrophilic polymer in the innermost layer has a higher number average molecular weight than the hydrophilic polymer in other hydrophilic polymer layers.

The Mn of the hydrophilic polymer in the innermost layer is preferably 5000 or more, more preferably 10000 or more, still more preferably 15000 or more, while it is preferably 200000 or less, more preferably 150000 or less, still more preferably 120000 or less. When the Mn is within the range indicated above, the advantageous effect tends to be better achieved.

The Mn of the hydrophilic polymer in other hydrophilic polymer layers is preferably 5000 or more, more preferably 6000 or more, still more preferably 7000 or more, while it is preferably 30000 or less, more preferably 25000 or less, still more preferably 20000 or less. When the Mn is within the range indicated above, the advantageous effect tends to be better achieved. Desirably, the Mn of the hydrophilic polymer in the hydrophilic polymer layer that is farthest from the surface of the glass substrate in substantially the vertical direction (hereinafter, also referred to as outermost surface layer) among the above-described other hydrophilic polymer layers is within the range indicated above.

When the hydrophilic polymer in the innermost layer among the two or more hydrophilic polymer layers has a higher number average molecular weight than the hydrophilic polymer in the above-described other hydrophilic polymer layers, the Mn of the hydrophilic polymer in the innermost layer is preferably 30000 or more, more preferably 40000 or more, still more preferably 60000 or more, while it is preferably 200000 or less, more preferably 150000 or less, still more preferably 120000 or less. When the Mn is within the range indicated above, the advantageous effect tends to be better achieved.

Herein, the number average molecular weight (Mn) can be determined by gel permeation chromatography (GPC) (GPC-8000 series available from Tosoh Corporation, detector: differential refractometer, column: TSKgel Super-Multipore HZ-M available from Tosoh Corporation) and calibrated with polystyrene standards.

The thickness of the two or more hydrophilic polymer layers as a whole (the total thickness of the two or more hydrophilic polymer layers) is preferably 10 to 1000 nm, more preferably 30 to 700 nm, still more preferably 50 to 350 nm. When the thickness is within the range indicated above, the advantageous effect tends to be better achieved. Further, low adsorption of normal proteins and cells and selective capture of cancer cells can be expected.

The thickness of the two or more hydrophilic polymer layers as a whole can be measured by the method described later in EXAMPLES.

In order to better achieve the advantageous effect, when the hydrophilic polymer in the innermost layer among the two or more hydrophilic polymer layers has a higher number average molecular weight than the hydrophilic polymer in the above-described other hydrophilic polymer layers, the thickness of the innermost layer is desirably not more than the thickness of the other hydrophilic polymer layers as a whole (total thickness excluding the thickness of the innermost layer). The ratio "thickness of the innermost layer/thickness of the hydrophilic polymer layers as a whole" is preferably 0.10 to 0.50, more preferably 0.10 to 0.40. The thickness of each hydrophilic polymer layer can be measured by the method described later in EXAMPLES.

The surface of the outermost surface layer among the two or more hydrophilic polymer layers at least partially (partially or entirely) has a water contact angle of preferably 60° or less, more preferably 50° or less. The lower limit is not limited, and a smaller angle is better.

The two or more hydrophilic polymer layers each may be formed by known methods such as (1) a method of introducing a hydrophilic polymer solution or dispersion in which the hydrophilic polymer is dissolved or dispersed in a solvent onto the surface of the glass substrate or the surface of a hydrophilic polymer layer formed in advance (depressed portions, etc. of the substrate), optionally followed by retaining or drying for a predetermined time and (2) a method of applying (spraying) the hydrophilic polymer solution or dispersion onto the surface of the glass substrate or the surface of a hydrophilic polymer layer formed in advance (depressed portions, etc. of the substrate), optionally followed by retaining or drying for a predetermined time. A polymer-coated glass substrate including two or more hydrophilic polymer layers on the entire or partial surface of the glass substrate can be produced by forming the innermost layer on the glass substrate, optional one or more hydrophilic polymer layers other than the outermost surface layer, and the outermost surface layer by such known methods. Then, the polymer-coated glass substrate may be combined with other components as needed to prepare a device capable of, for example, capturing, culturing, and/or analyzing specific cells such as cancer cells.

Conventionally known materials or methods are applicable as the solvent, introduction method, application

(spraying) method, etc.

The duration of retention/drying in the method (1) or (2) may be appropriately selected depending on the size of the glass substrate, the type of liquid introduced, etc. The duration of the retention is preferably 10 seconds to 10 hours, more preferably 1 minute to 5 hours, still more preferably 5 minutes to 2 hours. The drying is preferably performed at room temperature (about 23°C) to 80°C, more preferably at room temperature to 60°C. Moreover, the drying may be performed under reduced pressure. Further, after a predetermined time of retention, the excess hydrophilic polymer solution or dispersion may be discharged as appropriate before drying.

The solvent may be any solvent that can dissolve a hydrophilic polymer and may be appropriately selected depending on the hydrophilic polymer used. For example, the solvent may be water, an organic solvent, or a mixture thereof. Examples of the organic solvent include alcohols such as methanol, ethanol, n-propanol, i-propanol, and methoxypropanol; ketones such as acetone and methyl ethyl ketone; tetrahydrofuran, acetonitrile, ethyl acetate, and toluene. These may be used alone or in combinations of two or more.

The concentration of the hydrophilic polymer solution or dispersion is not limited and may be appropriately selected in view of introduction, application, or spraying properties, productivity, etc. The hydrophilic polymer concentration of the hydrophilic polymer solution or dispersion (100% by mass) is preferably 0.01 to 10.0% by mass, more preferably 0.10 to 5.0% by mass.

Of the two or more hydrophilic polymer layers, the outermost surface layer may have a scaffold protein adsorbed onto the outermost surface thereof. The adsorption of a scaffold protein to the surface of the outermost polymer layer can improve capture of specific cells such as cancer cells.

Herein, the term "scaffold protein" refers to a protein that has the function of promoting selective capture of the specific cells, such as a protein that has the function of specifically binding to a protein appearing on the surface of the specific cells. The term also refers to a protein that has the function of interacting with (e.g., adsorbing to, binding to, or associating with) the hydrophilic polymer, such as a protein which may adsorb to the hydrophilic polymer to mediate the function of promoting selective capture of the specific cells to the surface of the hydrophilic polymer.

The scaffold protein desirably has an arginine-glycine-aspartate (RGD) sequence. Suitable examples of the scaffold protein include fibronectin.

The scaffold protein may be adsorbed onto the outermost layer by any known method. For example, the scaffold protein may be adsorbed onto the outermost layer by bringing the outermost layer into contact with a buffer solution (e.g., phosphate buffered saline (PBS)) of the scaffold protein by a known method, and leaving them at a predetermined temperature for a predetermined time, optionally followed by washing. The temperature and duration may be selected as appropriate, and may be, for example, about 10°C to 60°C and about 0.1 to 24 hours, respectively.

From the standpoint of adsorbing the scaffold protein onto the outermost layer, it is suitable to use, for example, a solution or dispersion having a scaffold protein concentration preferably adjusted to 0.5 to 500 ug/ml, more preferably 1 to 250 ug/ml. When the scaffold protein concentration is adjusted within the range indicated above, excellent capture of specific cells such as cancer cells can be achieved. Here, the range indicated above is also desirable for the fibronectin concentration.

In the polymer-coated glass substrate, the surface of the two or more hydrophilic polymer layers desirably has a low elastic modulus in water or an aqueous solution.

Specific cells such as cancer cells are known to be generally softer than normal cells such as blood cells. This is related to the fact that when specific cells such as cancer cells metastasize, they deform their cell shape greatly and move through gaps. Thus, normal cells such as blood cells, which are hard and less deformable, are less likely to be captured by a polymer-coated glass substrate that is coated with a hydrophilic polymer having a soft surface. In contrast, specific cells such as cancer cells that have acquired deformability tend to be captured by a polymer-coated glass substrate having a soft surface. The two or more hydrophilic polymer layers on the glass substrate can form a hydrophilic polymer layer having a high flat smoothness and a low elastic modulus (high flexibility), thereby achieving excellent capture of specific cells such as cancer cells.

In order to reduce the capture of normal cells such as blood cells and selectively capture specific cells such as cancer cells, the elastic modulus is preferably 1.20 MPa or less, more preferably 0.80 MPa or less, still more preferably 0.50 MPa or less, particularly preferably 0.30 MPa or less. The lower limit is not limited and is preferably 0.01 MPa or more, more preferably 0.05 MPa or more, still more preferably 0.07 MPa or more.

The elastic modulus in water or an aqueous solution can be controlled by changing the molecular weight of the hydrophilic polymer in the two or more hydrophilic polymer layers or the film thickness. Specifically, the elastic modulus tends to increase as the molecular weight of the hydrophilic polymer increases or as the film thickness of the hydrophilic polymer layer increases.

Herein, the term "elastic modulus in water or an aqueous solution" refers to the elastic modulus in water or an aqueous solution measured with an atomic force microscope (AFM), unless otherwise stated.

An atomic force microscope (AFM) is a type of scanning probe microscope which detects the force acting between the atoms on the sample and the atoms on the probe. The probe is attached to the tip of the cantilever (cantilever

spring). The force (amount of deflection) acting on the cantilever can be measured while changing the distance between the sample and the probe to obtain a curve (force curve) plotting the relationships between them. Analysis of the force curve can determine the elastic modulus (hardness) of the surface of the sample at the nano scale. The method for determining the elastic modulus of the surface of the sample by the force curve analysis is known to those skilled in the art. The elastic modulus can be determined by such a known method.

For example, the elastic modulus may be calculated from the force curve by fitting the force curve according to the Johnson-Kendall-Roberts (JKR) theory to calculate the elastic modulus. According to the JKR theory, the force F applied to the cantilever and the amount of deformation δ of the sample are expressed by the following equations (1) and (2) with w representing the adhesion energy.

$$F = \frac{a^3}{R} - \sqrt{6\pi K w a^3} \qquad (1)$$

$$\delta = \frac{a^2}{3R} + \frac{2F}{3aK} \qquad (2)$$

In the equations, a denotes the radius of a contact line between the probe and the sample, R denotes the radius of curvature of the probe tip, and K denotes the elastic coefficient.

The elastic modulus can be determined from the F-δ curve obtained by the force curve analysis and the fitting using the equations (1) and (2).

Here, the elastic modulus in water or an aqueous solution specifically refers to the value measured as follows.

The elastic modulus in water or an aqueous solution can be measured with an AFM by dropping water or an aqueous solution on the surface of the sample to form a droplet (convex meniscus).

Suitable examples of the aqueous solution include phosphate buffered saline (PBS).

Then, the elastic modulus of the sample (the surface of the two or more hydrophilic polymer layers) may be determined, for example, by scanning the surface of the sample within a predetermined range to acquire force curves at multiple points within the predetermined range, determining the elastic moduli from the force curves, and calculating the average of the elastic moduli.

In the polymer-coated glass substrate, two or more hydrophilic polymer layers are formed on the surface of a glass substrate, whereby the irregularity of the substrate surface can be controlled. Thus, a hydrophilic polymer layer having a low surface roughness and a high flat smoothness can be formed. The hydrophilic polymer layer enables excellent capture of specific cells such as cancer cells. The surface of the formed two or more hydrophilic polymer layers has a low elastic modulus to provide much better capture of specific cells. Therefore, the number of specific cells in sampled blood or biological fluid can be determined by counting the number of captured specific cells, and the determined number can be used to evaluate the cancer-treating effect, etc. Moreover, the captured specific cells may be cultured and then used to determine the effects of drugs such as anticancer drugs or to screen anticancer drugs.

EXAMPLES

The present invention is specifically described below based on examples, but the present invention is not limited to the examples.

<Production of hydrophilic polymer>

(Production of polymer 1)

Using a solution of azobisisobutyronitrile (AIBN) in toluene (1.25 mg/g), 2-methoxyethyl acrylate (25 wt% in toluene) was thermally polymerized at 60°C for seven hours to produce poly(2-methoxyethyl acrylate) (PMEA, Mn: 16000).

(Production of polymer 2)

Using a solution of azobisisobutyronitrile (AIBN) in methanol (1.25 mg/ml), 2-methoxyethyl acrylate (50 wt% in methanol) was thermally polymerized at 60°C for seven hours to produce poly(2-methoxyethyl acrylate) (PMEA, Mn:

80000).

(Production of polymer 3)

Using a solution of azobisisobutyronitrile (AIBN) in methanol (1.25 mg/ml), 2-methoxyethyl acrylate (75 wt% in methanol) was thermally polymerized at 60°C for seven hours to produce poly(2-methoxyethyl acrylate) (PMEA, Mn: 120000).

<Production of polymer-coated glass substrate>

(Example 1)

An amount of 80 ul of a 0.125% solution of the PMEA (Mn: 16000) produced in the production of polymer 1 in methanol was introduced into one well of a slide chamber (two-well type) (uncoated, available from Matsunami Glass Ind., Ltd., bottom face: soda lime glass, water contact angle: 69.7°, average thickness: 1.35 μm), immediately followed by vacuum drying in an oven at 40°C for five minutes.

After cooling to room temperature, 80 ul of a 0.125% solution of the PMEA (Mn: 16000) produced in the production of polymer 1 in methanol was introduced onto the PMEA coating layer, immediately followed by vacuum drying in an oven at 40°C for five minutes. Thereby, a polymer-coated glass substrate was produced.

(Examples 2 to 7, Comparative Examples 1 to 3)

A polymer-coated glass substrate was produced as in Example 1 using the polymer 1, 2, or 3 (Mn), except for changing the concentration of the polymer 1, 2, or 3 in the methanol solution and the amount of the methanol solution introduced according to Table 1.

The PMEA as the polymer 2 (Mn: 80000) or the polymer 3 (Mn: 120000) was difficult to dissolve in methanol and was therefore warmed to 40°C and dissolved to prepare a polymer solution.

In Comparative Examples 1 to 3, only one hydrophilic polymer layer was formed.

The polymer-coated glass substrates produced in the examples and comparative examples were analyzed for occurrence of white turbidity of the polymer layer, the elastic modulus of the surface in phosphate buffered saline (PBS) with an AFM, and the thickness of the polymer layer. Table 1 shows the results. FIG. 1 shows pictures of glass substrates produced in Example 4 and Comparative Example 1.

[Occurrence of white turbidity of polymer layer]

After two hours from the coating, the surface was visually observed.

Occurrence of white turbidity indicates that the surface has a large surface roughness. The surface with less white turbidity has a higher flat smoothness.

[Elastic modulus of surface]

Phosphate buffered saline was dropped on the surface of the hydrophilic polymer layer of each polymer-coated glass substrate to form a droplet (convex meniscus). The elastic modulus of the surface was measured by the following method using the device (AFM) described below. The determined elastic modulus was defined as the elastic modulus measured in water or an aqueous solution.

Here, in the measurement of the elastic modulus, the obtained force curve was analyzed based on the JKR contact theory to determine the elastic modulus.

<Method for measuring elastic modulus>

Device (AFM): MFP-3D-SA available from Oxford Instruments
Measurement mode: AFM force curve mapping
Cantilever: material Si, radius of curvature of tip R = 150 nm, spring constant 0.67 N/m
Measurement range: scan of 20 μm × 20 um, calculation of elastic modulus by 2-point JKR method
Scanning rate: 1 Hz
Measurement environment: in PBS

Measurement temperature: 23°C

The elastic modulus of the surface measured as above was evaluated by the following criteria. A lower elastic modulus indicates better capture of specific cells such as cancer cells.

Low: 0.2 MPa or less
Slightly high: 0.4 to 1 MPa
High: more than 1 MPa

[Thickness of two or more hydrophilic polymer layers as a whole (total thickness of two or more hydrophilic polymer layers)]

A cross section of the hydrophilic polymer layer was measured (photographed) using TEM at an acceleration voltage of 15 kV and a magnification of 1000 times to determine the total thickness (total film thickness) of the hydrophilic polymer layers.

[Thickness (relative value) of each polymer layer]

The relative thickness value was expressed as a product of the concentration of the polymer solution and the amount of the polymer solution introduced.

[Table 1]

| | | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|
| First layer (Innermost layer) | Polymer type | PMEA | PMEA | PMEA | PMEA | PMEA | PMEA | PMEA | PMEA | PMEA | PMEA |
| | Mn of polymer | 16000 (Polymer 1) | 16000 (Polymer 1) | 120000 (Polymer 3) | 120000 (Polymer 3) | 120000 (Polymer 3) | 80000 (Polymer 2) | 80000 (Polymer 2) | 16000 (Polymer 1) | 80000 (Polymer 2) | 120000 (Polymer 3) |
| | Concentration of polymer solution | 0.1250% | 0.1250% | 0.1250% | 0.0625% | 0.0375% | 0.0625% | 0.0375% | 0.2500% | 0.2500% | 0.2500% |
| | Amount of polymer solution introduced | 80 µl | 90 µl | 70 µl | 70 µl | 80 µl | 70 µl | 80 µl | 80 µl | 80 µl | 80 µl |
| | Thickness of first layer (relative value) | 10 | 11.25 | 8.75 | 4.375 | 3 | 4.375 | 3 | 20 | 20 | 20 |
| Second layer (Outermost surface layer) | Polymer type | PMEA | PMEA | PMEA | PMEA | PMEA | PMEA | PMEA | - | - | - |
| | Mn of polymer | 16000 (Polymer 1) | 16000 (Polymer 1) | 16000 (Polymer 1) | 16000 (Polymer 1) | 16000 (Polymer 1) | 16000 (Polymer 1) | 16000 (Polymer 1) | - | - | - |
| | Concentration of polymer solution | 0.125% | 0.125% | 0.125% | 0.1875% | 0.2125% | 0.125% | 0.2125% | - | - | - |
| | Amount of polymer solution introduced | 80 µl | 70 µl | 90 µl | 90 µl | 80 µl | 90 µl | 80 µl | - | - | - |
| | Thickness of second layer (relative value) | 10 | 8.75 | 11.25 | 16.875 | 17 | 11.25 | 17 | - | - | - |
| Thickness of innermost layer/ Thickness of hydrophilic polymer layers as a whole | | 0.50 | 0.56 | 0.44 | 0.21 | 0.15 | 0.28 | 0.15 | - | - | - |
| Total thickness of hydrophilic polymer layers (TEM) (nm) | | 80 | 80 | 81 | 85 | 82 | 84 | 81 | 80 | 82 | 85 |
| White turbidity of polymer layer | | Not occurred | Not occurred | Not occurred | Not occurred | Not occurred | Not occurred | Not occurred | Occurred one out of every three times | Not occurred | Not occurred |

(continued)

| | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|
| Elastic modulus of surface | Low | Low | Low | Low | Low | Low | Low | Low | High | High |

In Comparative Example 1 in which only one layer of PMEA having Mn of 16000 was formed, white turbidity occurred one out of every three times. In contrast, in Examples 1 and 2 in which two layers of PMEA having Mn of 16000 were formed, white turbidity did not occur, and the elastic modulus was low.

In Examples 3 to 5 in which the Mn in the first layer was increased to 120000 and Examples 6 and 7 in which the Mn in the first layer was increased to 80000, white turbidity did not occur, and the elastic modulus was low. In contrast, in Comparative Examples 2 and 3 in which only one layer of PMEA having Mn of 80000 or 120000 was formed, the elastic modulus of the surface was high.

Moreover, when the first layer had a higher Mn than the second layer, and also the thickness of the first layer was not more than a half the total thickness of the hydrophilic polymer layers, white turbidity did not occur, and the elastic modulus was low.

Exemplary embodiments of the present invention include the following.

Embodiment 1. A polymer-coated glass substrate including

a glass substrate and
two or more hydrophilic polymer layers on a surface of the glass substrate.

Embodiment 2. The polymer-coated glass substrate according to Embodiment 1,
wherein the two or more hydrophilic polymer layers include the same hydrophilic polymer or different hydrophilic polymers.

Embodiment 3. The polymer-coated glass substrate according to Embodiment 1 or 2,

wherein the two or more hydrophilic polymer layers include the same hydrophilic polymer, and
the hydrophilic polymer in the hydrophilic polymer layer on the surface of the glass substrate among the two or more hydrophilic polymer layers has a number average molecular weight equal to or higher than that of the hydrophilic polymer in other hydrophilic polymer layers.

Embodiment 4. The polymer-coated glass substrate according to any combination with any one of Embodiments 1 to 3,

wherein the two or more hydrophilic polymer layers include the same hydrophilic polymer,
the hydrophilic polymer in the hydrophilic polymer layer on the surface of the glass substrate among the two or more hydrophilic polymer layers has a higher number average molecular weight than the hydrophilic polymer in other hydrophilic polymer layers, and
a thickness of the hydrophilic polymer layer on the surface of the glass substrate is not more than a thickness of other hydrophilic polymer layers as a whole.

Embodiment 5. The polymer-coated glass substrate according to Embodiment 3 or 4,
wherein the hydrophilic polymer in other hydrophilic polymer layers has a number average molecular weight of 30000 or less.

Embodiment 6. The polymer-coated glass substrate according to any combination with any one of Embodiments 1 to 5,
wherein the two or more hydrophilic polymer layers each include at least one selected from the group consisting of hydrophilic polymers represented by the following formula (I):

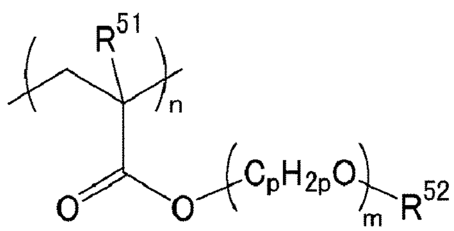

(I)

wherein $R^{51}$ represents a hydrogen atom or a methyl group;
$R^{52}$ represents an alkyl group; p represents 1 to 8; m represents 1 to 5; and n represents the number of repetitions.

Embodiment 7. The polymer-coated glass substrate according to any combination with any one of Embodiments 1 to 5,
wherein the two or more hydrophilic polymer layers each include a copolymer of at least one hydrophilic monomer

selected from the group consisting of compounds represented by the following formula (II) with a different monomer:

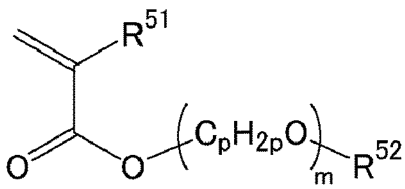

(II)

wherein $R^{51}$ represents a hydrogen atom or a methyl group;
$R^{52}$ represents an alkyl group; p represents 1 to 8; and m represents 1 to 5.

Embodiment 8. The polymer-coated glass substrate according to any combination with any one of Embodiments 1 to 7,
wherein a thickness of the two or more hydrophilic polymer layers as a whole is 10 to 1000 nm.
Embodiment 9. The polymer-coated glass substrate according to any combination with any one of Embodiments 1 to 8,
wherein a scaffold protein is adsorbed onto a surface of an outermost surface layer among the two or more hydrophilic polymer layers.
Embodiment 10. The polymer-coated glass substrate according to Embodiment 9,
wherein the scaffold protein is fibronectin.

## Claims

**1.** A polymer-coated glass substrate comprising

a glass substrate and
two or more hydrophilic polymer layers on a surface of the glass substrate.

**2.** The polymer-coated glass substrate according to claim 1,
wherein the two or more hydrophilic polymer layers comprise the same hydrophilic polymer or different hydrophilic polymers.

**3.** The polymer-coated glass substrate according to claim 1 or 2,

wherein the two or more hydrophilic polymer layers comprise the same hydrophilic polymer, and
the hydrophilic polymer in the hydrophilic polymer layer on the surface of the glass substrate among the two or more hydrophilic polymer layers has a number average molecular weight equal to or higher than that of the hydrophilic polymer in other hydrophilic polymer layers.

**4.** The polymer-coated glass substrate according to any one of claims 1 to 3,

wherein the two or more hydrophilic polymer layers comprise the same hydrophilic polymer,
the hydrophilic polymer in the hydrophilic polymer layer on the surface of the glass substrate among the two or more hydrophilic polymer layers has a higher number average molecular weight than the hydrophilic polymer in other hydrophilic polymer layers, and
a thickness of the hydrophilic polymer layer on the surface of the glass substrate is not more than a thickness of other hydrophilic polymer layers as a whole.

**5.** The polymer-coated glass substrate according to claim 3 or 4,
wherein the hydrophilic polymer in other hydrophilic polymer layers has a number average molecular weight of 30000 or less.

**6.** The polymer-coated glass substrate according to any one of claims 1 to 5,
wherein the two or more hydrophilic polymer layers each comprise at least one selected from the group consisting of hydrophilic polymers represented by the following formula (I):

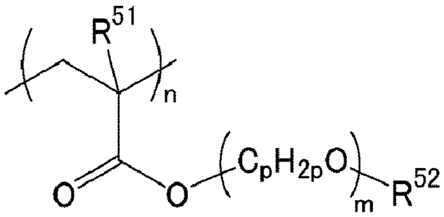

(I)

wherein $R^{51}$ represents a hydrogen atom or a methyl group;
$R^{52}$ represents an alkyl group; p represents 1 to 8; m represents 1 to 5; and n represents the number of repetitions.

**7.** The polymer-coated glass substrate according to any one of claims 1 to 5, wherein the two or more hydrophilic polymer layers each comprise a copolymer of at least one hydrophilic monomer selected from the group consisting of compounds represented by the following formula (II) with a different monomer:

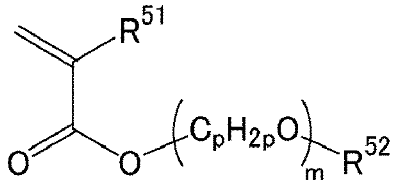

(II)

wherein $R^{51}$ represents a hydrogen atom or a methyl group;
$R^{52}$ represents an alkyl group; p represents 1 to 8; and m represents 1 to 5.

**8.** The polymer-coated glass substrate according to any one of claims 1 to 7, wherein a thickness of the two or more hydrophilic polymer layers as a whole is 10 to 1000 nm.

**9.** The polymer-coated glass substrate according to any one of claims 1 to 8, wherein a scaffold protein is adsorbed onto a surface of an outermost surface layer among the two or more hydrophilic polymer layers.

**10.** The polymer-coated glass substrate according to claim 9, wherein the scaffold protein is fibronectin.

FIG. 1

Example 4

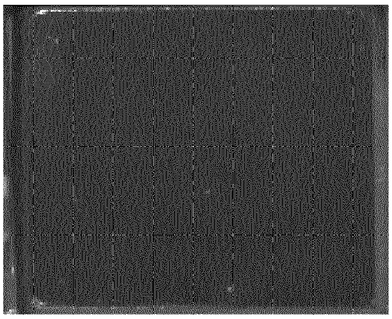

Comparative Example 1

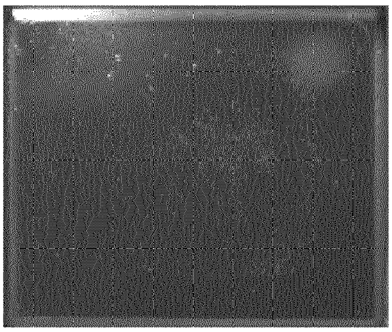

Europäisches Patentamt
European Patent Office
Office européen des brevets

# EUROPEAN SEARCH REPORT

Application Number
EP 24 15 0092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AMORIM SARA ET AL: "Multilayer platform to model the bioactivity of hyaluronic acid in gastric cancer", MATERIALS SCIENCE AND ENGINEERING C, ELSEVIER SCIENCE S.A, CH, vol. 119, 9 October 2020 (2020-10-09), XP086402170, ISSN: 0928-4931, DOI: 10.1016/J.MSEC.2020.111616 [retrieved on 2020-10-09] * par. 2 "Materials and methods" * * figure 1 * * left col, second full paragraph.; page 4 * ----- | 1-3,8 | INV. C03C17/32 B32B17/10 C03C17/34 C09D133/14 G01N33/00 |
| X | BOUDOU THOMAS ET AL: "Internal Composition versus the Mechanical Properties of Polyelectrolyte Multilayer Films: The Influence of Chemical Cross-Linking", LANGMUIR, vol. 25, no. 24, 3 September 2009 (2009-09-03), pages 13809-13819, XP93142498, US ISSN: 0743-7463, DOI: 10.1021/la9018663 * par. "Materials and methods" * * figures 1, 3B * ----- -/-- | 1-4,8 | TECHNICAL FIELDS SEARCHED (IPC) C03C B32B G01N C08F C09D |

The present search report has been drawn up for all claims

| Place of search | Date of completion of the search | Examiner |
|---|---|---|
| Munich | 19 March 2024 | Omegna, Anna |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 15 0092

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KUMAR THARAGAN ET AL: "Multi-layer assembly of cellulose nanofibrils in a microfluidic device for the selective capture and release of viable tumor cells from whole blood", NANOSCALE, vol. 12, no. 42, 5 November 2020 (2020-11-05), pages 21788-21797, XP093141187, United Kingdom ISSN: 2040-3364, DOI: 10.1039/D0NR05375A Retrieved from the Internet: URL:https://pubs.rsc.org/en/content/articlepdf/2020/nr/d0nr05375a> * par. 2. "Experimental methods" * * figure 1 * * par. 2.5, par. 3.1 * ----- | 1-4,8 | |
| X | US 2021/372895 A1 (MINAGAWA YASUHISA [JP]) 2 December 2021 (2021-12-02) * examples * * paragraph [0072] - paragraph [0083] * * paragraph [0088] - paragraph [0090] * ----- | 1-10 | TECHNICAL FIELDS SEARCHED (IPC) |

The present search report has been drawn up for all claims

| Place of search | Date of completion of the search | Examiner |
|---|---|---|
| Munich | 19 March 2024 | Omegna, Anna |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 15 0092

This annex lists the patent family members relating to the patent documents cited in the above-mentioned European search report. The members are as contained in the European Patent Office EDP file on
The European Patent Office is in no way liable for these particulars which are merely given for the purpose of information.

19-03-2024

| Patent document cited in search report | Publication date | Patent family member(s) | Publication date |
|---|---|---|---|
| US 2021372895 A1 | 02-12-2021 | CN 113735457 A | 03-12-2021 |
| | | EP 3915957 A1 | 01-12-2021 |
| | | JP 2021187907 A | 13-12-2021 |
| | | US 2021372895 A1 | 02-12-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005523981 T